# EUROPÄISCHE PATENTSCHRIFT

(11) **EP 0 501 206 B1**
(45) Veröffentlichungstag und Bekanntmachung des Hinweises auf die Patenterteilung: **28.08.1996**
(21) Anmeldenummer: 92102062.4
(22) Anmeldetag: 07.02.1992
(51) Int. Cl.: C07H 15/203, A61K 31/70, A61K 31/11, A61K 31/12, A61K 31/19, A61K 31/235

(54) **Phenonverbindungen, Verfahren zu ihrer Herstellung und diese enthaltende pharmazeutische Zubereitungen**
Phenone compounds, process for their preparation and their pharmaceutical preparations
Dérivés de phénones, procédé pour leur préparation et leurs compositions pharmaceutiques

(30) Priorität: 26.02.1991 DE 4106028; 24.01.1992 DE 4201942
(43) Veröffentlichungstag der Anmeldung: 02.09.1992
(73) Patentinhaber: Plantamed Arzneimittel GmbH, D-92318 Neumarkt (DE)
(72) Erfinder: Wagner, Hildebert, Prof. Dr. Dr., Inst. für pharm., W-8000 München 2 (DE); Stuppner, Hermann, Dr., Universität Innsbruck, A-6020 Innsbruck (AT); Dorsch, Walter, Prof. Dr., W-8000 München 90 (DE); Antus, Sandor, Dr., H-1521 Budapest (HU)
(74) Vertreter: Sandmair, Kurt, Dr. Dr.

(56) Entgegenhaltungen:
- EP-A- 0 051 819
- EP-A- 0 276 065
- DE-A- 2 351 385
- FR-A- 2 584 928
- CARBOHYDRATE RESEARCH, Bd.198, 1990, AMSTERDAM NL Seiten 223 - 234 D. DELAY ET AL 'NEW SYNTHESES OF PLANT ARYL GLYCOSIDES AS POTENTIAL GENE INDUCERS'
- CHEMICAL ABSTRACTS, vol. 82, no. 9, 3. März 1975, Columbus, Ohio, US; abstract no. 58057, L. REICHEL ET AL 'Chemistry and Biochemistry of Plant Consitutents' Seite 604 ;Spalte 2 ;
- CHEMICAL ABSTRACTS, vol. 115, no. 19, 11. November 1991, Columbus, Ohio, US; abstract no. 197940d, B. 'T HART ET AL 'Antiarthritic activity of the newly developed neutrophil oxidative burst antagonist apocynin' Seite 45 ;Spalte 1 ;
- CHEMICAL ABSTRACTS, vol. 115, no. 21, 25. November 1991, Columbus, Ohio, US; abstract no. 223171, W. DORSCH ET AL 'Antiasthmatic effects of Picrorhiza kurroa: androsin prevents allergen- and PAF-induced bronchial obstruction in guinea pigs' Seite 64 ;Spalte 2 ;
- SUSAN BUDAVARI, EDITOR 'THE MERCK INDEX' 1989 , MERCK & CO., INC. , RAHWAY, N.J., U.S.A.
- DATABASE WPI Week 8925, Derwent Publications Ltd., London, GB; AN 89-182773 & JP-A-1 121 236 (TERUMO CORP)
- PLANTA MEDICA, Bd.55, Nr.5, 1989 Seiten 467 - 469 H. STUPPNER UND H. WAGNER 'Minor Iridoid and Phenol Glycosides of Picrorhiza kurroa'
- CHEMICAL ABSTRACTS, vol. 101, no. 24, 10. Dezember 1984, Columbus, Ohio, US; abstract no. 216449e, 'Analgesics containing salicylic acid derivatives' Seite 319 ;Spalte 2 ;
- Week 8920, Derwent Publications Ltd., London, GB; AN 83-62093K & JP-A-58 083 619 (KUREHA CHEM IND KK) 19. Mai 1983
- Week 8938, Derwent Publications Ltd., London, GB; AN 89-273365 & JP-A-1 197 436 (TERUMO CORP) 1. Februar 1988
- Week 8436, Derwent Publications Ltd., London, GB; AN 84-223393 & RO-A-83 317 (INTR PLAFAR) 30. April 1984
- PHARMAZIE, Bd.26, 1971 Seiten 434 - 436 H. THIEME UND H.-J. WINKLER 'Über Vorkommen und Akkumulation von Phenolglykosiden in der Familie der Primulaceen'
- CHEMICAL ABSTRACTS, vol. 85, no. 9, 30. August 1976, Columbus, Ohio, US; abstract no. 59569a, S. Z. IVANOVA ET AL 'Glycosides of acetophenones from Picea obovata needles' Seite 291 ;Spalte 1 ;
- CHEMICAL ABSTRACTS, vol. 97, no. 12, 20. September 1982, Columbus, Ohio, US; abstract no. 98364x, 'Hypotensive pharmaceuticals' Seite 401 ;Spalte 1 ;

## Beschreibung

Die Erfindung betrifft bestimmte neue Phenonverbindungen, ein Verfahren zu ihrer Herstellung sowie neue Verwendungen von als Verbindungen bekannter Phenone in pharmazeutischen Zubereitungen, wie sie in den Patentansprüchen gekennzeichnet sind.

Die pharmazeutischen Zubereitungen sind zur Prophylaxe und Therapie von allergischen Erkrankungen insbesondere der Haut und, Asthma-Erkrankungen brauchbar. Der Anwendungsbereich entspricht dem der bisher wirksamsten Antiphlogistika, nämlich den Corticoiden.

Unter dem nachstehend verwendeten Begriff der Entzündung wird allgemein eine Reaktion des Organismus und seiner Gewebe gegen verschiedenartige schädigende Reize verstanden. Unter schädigenden Reizen sind exogene und auch endogene Reize, wie z.B. Gewebsverletzungen, das Eindringen von Fremdkörpern, chemische Stoffe, bakterielle Toxine, Allergene, Immunkomplexe, Mikroorganismen, krankhafte Stoffwechselprodukte sowie Zerfallsprodukte von Geschwülsten zu verstehen. Eng mit dem Entzündungsgeschehen verbunden sind die klassischen Symptome Schmerz und Fieber.

Es ist seit längerem bekannt, daß bestimmte körpereigene Substanzen, die sogenannten Mediatoren, eng mit dem Entzündungsablauf verbunden sind. Diese Mediatoren, denen eine außerordentlich große pathogenetische Bedeutung zukommt, werden durch das schädigende Ereignis (Noxe) aus den Körperzellen freigesetzt. Als wichtigste und bekannteste Mediatoren gelten Histamin, 5-HT (5-Hydroxytryptamin), Bradykinin, die Prostaglandine, Prostacycline, die Leukotriene, Thromooxane und der erst in neuerer Zeit charakterisierte Thrombozyten aktivierende Faktor (PAF; engl.: platelet activating factor).

Diese und weitere, im einzelnen nicht näher spezifizierte, Mediatoren wirken außerordentlich stark auf die Kontraktion der glatten Muskulatur, führen zu Herzfunktionsstörungen und beeinträchtigen die Integrität von Blutgefäßen und Schleimhäuten, wie z.B. die des Bronchialsystems. Sie bewirken außerdem die Aggregation von Thrombozyten und polymorphkernigen Leukozyten mit den schwerwiegenden Folgen einer anaphylaktischen Atemwegsverengung, Blutdruckabfall, Herz-Arrythmien, Plasmaexudation, Gewebsödeme, Hömokonzentration, Thrombozytopenie, Leukozytopenie, Verklumpung der Thrombozyten und polymorphkernigen Leukozyten in den Lungenkapillaren sowie schwerste Atmungsstörungen und Kreislaufkollaps.

Aufgrund ihres breiten pharmakologischen Wirkungsspektrums, ihrer großen Verbreitung im Organismus, ihrer Bildung durch zahlreiche physikalische, chemische, pathologische, pathophysiologische und pharmakologische Einflüsse sowie aufgrund ihrer Beteiligung bei einer Vielzahl pathophysiologischer Abläufe sind die Mediatioren bzw. deren pharmakologische Beeinflussung von größter medizinischer Bedeutung (vgl. "The Pharmacological Basis of Therapeutics, Ed. Goodman and Gilman, 6. Auflage, 1980, McMillan Publishing Company).

Dem PAF-Faktor scheint in der Pathogenese entzündlicher und allergischer Vorgänge eine besondere Bedeutung zuzukommen.

PAF ist ein Glycerophosphocholin mit der chemischen Bezeichnung 1-0-Alkyl-2-acetyl-sn-glyceril-3-phosphorylcholin. Dieser Faktor wird von einer Reihe von Zellen, wie z.B. Makrophagen, basophilen und neutrophilen Granulozyten u.a. bei Aktivierung freigesetzt. Die Freisetzung von PAF führt in weiterer Folge zu den vorstehend beschriebenen pathologischen Zuständen und wahrscheinlich noch zu einer Reihe weiterer, bislang nicht völlig verstandener, pathologischer Symptome. PAF muß nicht direkt wirken, sondern kann seine Wirkung vielmehr über eine Stimulierung weiterer Mediatoren entfalten. Neuere Untersuchungen zeigten, daß PAF insbesondere eine wichtige Rolle in der Genese des klinischen Bronchialasthmas sowie bei anderen pathologischen Zuständen der Lunge, z.B. der obstruktiven Bronchitis, besitzt.

Neben seiner wichtigen Rolle bei der Genese des Bronchialasthmas und bei der Anaphylaxie ist PAF als hochpotenter Entzündungsmediator mit den bereits oben beschriebenen pathologischen Wirkungen anzusehen.

Eine Vielzahl der oben genannten Mediatoren, einschließlich des PAF, werden durch eine membrangebundene Phospholipase aus Phospholipiden der Zellmembran freigesetzt, wobei einerseits Arachidonsäure und andererseits eine PAF-Vorstufe gebildet wird.

Ausgehend von Arachidonsäure werden zwei Gruppen von Mediatoren gebildet:
i) durch das Enzym Cyclooxygenase die Prostaglandine einschließlich des Prostacyclins und des Thromboxans;
ii) durch das Enzym Lipoxygenase die offenkettigen Hydroperoxy- und Hydroxysäuren und insbesondere die Leukotriene.

Die PAF-Vorstufe wird durch eine Acetyltransferase in die aktive Verbindung überführt.

Pharmakologisch bedeutsam bei der Behandlung von Entzündungen sind zwei Gruppen von Wirksubstanzen; es handelt sich einmal um die sogenannten nicht-steroidalen Antiphlogistika, das sind Verbindungen und Derivate der Salicylsäure. Weitere Verbindungen mit bekannter antiphlogistischer Wirkung sind die Pyrazolonderivate, die para-Aminophenolderivate, die Indolderivate (z.B. Indomethacin) und Derivate der Propionsäure. Die pharmakologische Wirkung aller dieser Verbindungen beruht darauf, daß sie die Cyclooxygenase zu hemmen vermögen und damit die Synthese der Prostaglandine oder Thromboxane unterbinden.

Salicylsäure bzw. ihre Derivate und die weiteren Verbindungen der gesamten Klasse sind mit einer Reihe schwerer und schwerster Nebenwirkungen behaftet. So führt z. B. die längerdauernde Verabreichung von Salicylsäurederivaten zur Magen- und Darmulzeration. Ebenfalls bekannt sind die relative Unverträglichkeit der Pyrazolonderivate, die hepatotoxische Wirkung der para-Aminophenolderivate, die allgemeine Unverträglichkeit von Indomethacin sowie die ulzerative Wirkung der Propionsäurederivate.

Ein weiterer ebenfalls schwerwiegender Nachteil der nicht-steroidalen Antiphlogistika besteht darin, daß sie unter Umständen die pathologische Wirkung der Mediatoren verstärken, indem durch die Hemmung der Cyclooxygenase vermehrt Substrat für die Lipoxygenase und damit für die Bildung von Leukotrienen bereitgestellt wird.

Den nicht-steroidalen Antiphlogistika gegenüber stehen die steroidalen Antiphlogistika, das sind die Corticosteroide und ihre Derivate. Die antiphlogistische Wirkung der Corticosteroide beruht auf ihrer Fähigkeit, sowohl die Phospholipase als auch die Lipoxygenase zu hemmen, wodurch der gesamte Arachidonsäurestoffwechsel inhibiert wird. Nachteilig für die Therapie mit Corticosteroiden sind die fatalen Nebenwirkungen, von denen nur beispielhaft die folgenden erwähnt werden sollen: Ulcera duodeni oder ventriculi, Myopathie, Osteoporosen, psychische Störungen, erhöhte Infektionsanfälligkeit, subkapsuläre Katarakte und dergleichen mehr.

Neben den Corticosteroiden sind selektive Lipoxygenasehemer, wie z. B. Benoxaprofen, in Benutzung. Auch diese Substanzklasse ist mit schweren Nebenwirkungen behaftet wie z. B. tödlich verlaufende exfoliative Dermatitiden (Syndrom der verbrühten Haut).

In Chem. Abstr., 115 (1991), Nr. 197940d, wird die antiinflammatorische Wirkung des Abocynins mit der chemischen Strukturbezeichnung 1-(4-Hydroxy-3-methoxyphenyl)-ethanon beschrieben. Die antiphlogistische Wirkung wird in dieser Publikation anhand des Kollagen-Arthritismodells an Ratten demonstriert. In der japanischen Patentanmeldung JP-A-1197436 wird ein methyliertes Aryl-2,4-dihydroxyacetophenon mit antirheumatischer Wirkung und entsprechende Arzneipräparate beschrieben. In der EP-A-0 276 065 wird eine spezielle Gruppe von Hydroxyacetophenonen mit Substituenten in der 2- und 5-Stellung beschrieben. Diese Verbindungen werden zur Behandlung der Psoriasis, einer Hauterkrankung unbekannter Genese, und chronisch entzündlichen Darmerkrankungen (IBD) verwendet.

Gegenstand der Erfindung ist daher die Verwendung einer Verbindung der allgemeinen Formel 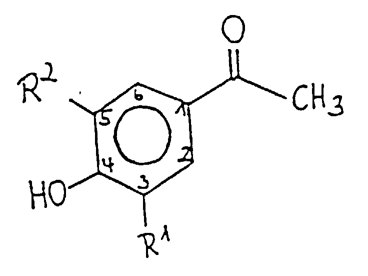 worin
R¹ = H, Cl, Br, I, Methyl, Hydroxy, Methoxy, Propoxy, Isopropoxy oder Ethoxy, und R² = H, Cl, Br, I, Methyl, Hydroxy, Methoxy, Propoxy, Isopropoxy oder Ethoxy darstellen, wobei R¹ und R² nicht gleichzeitig H bedeuten,
oder eines pharmazeutisch annehmbaren Ethers oder Esters davon in Kombination mit einem pharmazeutisch annehmbaren Träger zur Herstellung von Arzneimitteln zur Behandlung und/oder Prophylaxe des Asthma bronchiale, der obstruktiven Bronchitis oder allergischen Hauterkrankungen. Bevorzugt ist die Verwendung von Verbindungen, worin in der allgemeinen Formel R¹ Methyl und R² Wasserstoff, R¹ die Gruppe n-Propoxy und R² Wasserstoff, R₁ die Gruppe Isopropoxy und R² Wasserstoff, oder R¹ und R² jeweils die Methoxygruppe bedeuten.

Ein weiterer Erfindungsgegenstand sind Verbindungen der allgemeinen Formel 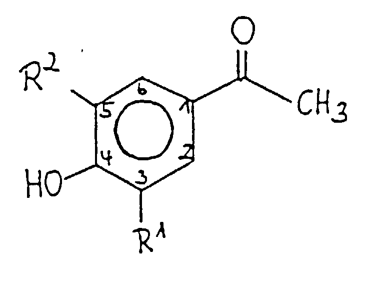 worin R¹ Methoxy und R² = Cl, Br oder I bedeuten,
sowie deren pharmazeutisch annehmbare Ester und Ether, sowie das Verfahren zur Herstellung dieser halogenierten Verbindungen, das dadurch gekennzeichnet ist, daß man ein Acetophenol der allgemeinen Formel 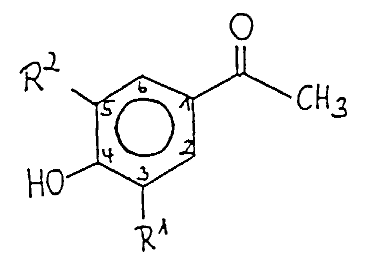 worin R¹ die Methoxygruppe und R² Wasserstoff bedeuten,
a) in einem Wasser/Methanol-Gemisch löst,
b) Natriumacetat hinzufügt und mit einer Lösung von Natriumhypochlorit oder Kaliumjodid und Jod (gasförmig) oder Kaliumbromid und Brom (gasförmig) für 1,5 bis 3 Stunden bei -60 °C bis 90 °C umsetzt,
c) das Reaktionsprodukt mit Chloroform ausschüttelt, mit Natriumthiosulfat wäscht und aus Essigsäure umkristallisiert und erforderlichenfalls zu dem gewünschten Ether oder Ester weiterverarbeitet.

Erfindungsgemäß werden ferner Arzneimittel geschaffen, die dadurch gekennzeichnet sind, daß sie eine oder mehrere halogenierte Verbindungen der vorstehenden allgemeinen Formel enthält. Ferner ist die Verwendung einer halogenierten Verbindung der vorstehenden allgemeinen Formel zur Herstellung von Arzneimitteln zur Behandlung und Prophylaxe von Asthma bronchiale, obstruktiver Bronchitis oder allergischen Erkrankungen.

Beispiele für Ethergruppen in Form von Glycosyleinheiten sind Glucosyl, Galaktosyl, Rhamnosyl und Arabinosyl.

Beispiele für die Substituenten an den Glykosyleinheiten sind Cinnamoyl-, Caffeoyl- und Benzoylreste.

Die Anmelderin hat nun überraschend gefunden, daß bestimmte Phenonverbindungen der nachstehend angegebenen allgemeinen Formel ausgzeichnete anti-phlogistische Wirkung bei gleichzeitiger Aktivität gegen Asthma und allergische Hauterkrankungen besitzen. Ein Teil dieser Verbindungen ist neu, wie weiter untenstehend im einzelnen definiert ist.

Die erfindungsgemäßen Verbindungen und die bekannten Phenonderivate zur erfindungsgemäßen Verwendung bzw. die entsprechenden pharmazeutischen Zubereitungen besitzen die wesentliche und wichtige Eigenschaft entzündliche Vorgänge, insbesondere PAF induzierte Effekte zu blockieren. Die erfindungsgemäßen Verbindungen der Formeln (I) und die Verbindungen der Formel (II) besitzen daher die Eigenschaft von steroidalen Antiphlogistika, ohne jedoch deren fatale Nebenwirkungen aufzuweisen und sind daher wertvoll zur Behandlung von Entzündungsvorgängen im weitesten Sinn, an deren Entstehung die bekannten Mediatoren, wie z. B. Prostaglandine, Histamin, PAF, Leukotriene und Thromboxane eine Rolle spielen.

Im Gegensatz zu den nicht-steroidalen Antiphlogistika, die nicht gegen die von Leukozyten ausgelösten Effekte von chronisch entzündlichen Erkrankungen wirken, da sie die Leukotrien-Bildung nicht inhibieren, sind die hier verwendeten Verbindungen aufgrund ihrer überraschenden, ausgezeichneten und vorteilhaften Wirkung auch in dieser Hinsicht den bekannten Verbindungen überlegen.

Die erfindungsgemäßen Verbindungen und die erfindungsgemäßen Zubereitungen erfüllen das Erfordernis für nicht-steroidale Antiphlogistika, die die Synthese der Leukotriene und Prostaglandine und Thromboxane inhibieren und den Effekten des PAF Faktors entgegenwirken. Sie sind frei von den mit den steroidalen oder nicht-steroidalen Antiphlogistika verbundenen Nachteilen.

Die erfindungsgemäßen Verbindungen bzw. die erfindungsgemäßen Zubereitungen besitzen bislang keine feststellbare Toxizität.

Die Verbindungen sowie ihre pharmazeutisch annehmbaren Zubereitungen können in niedrigen Dosen zur Erzielung einer therapeutischen entzündungshemmenden Wirkung verabreicht werden.

Die erfindungsgemäßen Arzneimittel die die vorstehend halogenierten Verbindungen beinhalten können zur Behandlung von Entzündungen der Gelenke, der Haut, der Schleimhäute sowie innerer Organe verwendet werden, unabhängig davon, ob die Entzündung durch Infektionserreger, immunologische Vorgänge oder Traumen hervorgerufen wurde. Hierbei sind insbesondere Entzündungsvorgänge des Bronchialsystems, wie Asthma bronchiale oder obstruktive Bronchitis besonders vorteilhaft zu behandelnde Indikationen. Weiterhin können die erfindungsgemäßen Arzneimittel zur Prophylaxe und Behandlung von Gefäß- und Herzerkrankungen verwendet werden, bei denen es wünschenswert erscheint, die Biosynthese von Entzündungsstoffen durch Thrombozyten zu inhibieren. Besonders vorteilhaft können die erfindungsgemäßen Arzneimittel zur Prophylaxe oder Behandlung aller PAF und/oder Leukotrien induzierten Phänomene und insbesondere zur Behandlung des Bronchialraums verwendet werden.

Die erforderliche Menge der zu verwendenden Verbindung (im folgenden als der aktive Bestandteil bezeichnet) im Arzneimittel für die gewünschte therapeutische Wirkung hängt von der jeweiligen Verbindung, der Verabreichungsart und von dem zu behandelnden Subjekt, sowie der jeweiligen Krankheit ab. Eine geeignete Dosis einer Verbindung zur Verabreichung an ein Säugetier, das an einer Entzündung, einem schmerzhaften oder fiebrigen Zustand, wie vorhin beschrieben, leidet, beträgt etwa 0,1 µg bis 500 mg des aktiven Bestandteils pro Kilogramm Körpergewicht. Im Falle einer systemischen Verabreichung kann die Dosis im Bereich von 0,5 bis 500 mg der aktiven Verbindung pro Kilogramm Körpergewicht, und die am meisten bevorzugte Dosis im Bereich von 0,5 bis 50 mg pro Kilogramm Körpergewicht, z.B. 5 bis 25 mg pro Kilogramm Körpergewicht, betragen, die mehrmals täglich, insbesondere zwei- oder dreimal täglich, verabreicht wird.

Im Falle einer topischen Verabreichung, z. B. auf die Haut oder Schleimhäute, kann die geeignete Dosis deutlich höher liegen; bei der inhalativen Verabreichung kann die Dosis jedoch deutlich unter der systemischen liegen.

Obwohl es grundsätzlich möglich ist, den aktiven Bestandteil alleine zu verabreichen, ist es vorzuziehen, daß der aktive Bestandteil in Form einer pharmazeutischen Formulierung, die eine Verbindung gemäß der allgemeinen Formel und einen dafür pharmazeutisch annehmbaren Trägerstoff enthält, verabreicht wird. Üblicherweise liegt der aktive Bestandteil in einer derartigen Formulierung in einer Konzentration von 0,1 bis 99,9 Gew.-% der Formulierung vor. Üblicherweise enthält eine Einzeldosis einer Formulierung zwischen 0,1 mg und 1 g des aktiven Bestandteils. Für die topische Verabreichung beträgt die Konzentration des aktiven Bestandteils vorzugsweise 1 bis 2 Gew.-% der Zubereitung, jedoch kann der aktive Bestandteil bis zu 10 Gew.-% ausmachen. Zubereitungen, die für eine nasale oder buccale Verabreichung vorgesehen sind, wie z.B. selbstzerstäubende Pulver, Sprays oder andere bekannte und übliche Vorrichtungen können 0,1 bis 20 Gew.-%, z.B. 2 Gew.-%, des aktiven Bestandteils enthalten.

Die erfindungsgemäßen Zubereitungen zur Verwendung in der Veterinär- als auch in der Humanmedizin enthalten den aktiven Bestandteil in Verbindung mit einem dafür pharmazeutisch annehmbaren Trägerstoff und gegebenenfalls weiteren therapeutisch aktiven Bestandteilen. Der Trägerstoff muß annehmbar in dem Sinne sein, daß er mit den anderen Bestandteilen der Formulierung kompatibel ist und keine nachteilige Wirkung auf den Empfänger der Formulierung besitzt.

Die Formulierungen liegen geeigneterweise in Form einer oralen, ophtalmologischen, rektalen, parenteralen (einschließlich subkutanen, intramuskulären und intravenösen), intraartikulären, topischen, nasalen oder buccalen Verarbreichungsform vor.

Die erfindungsgemäßen Formulierungen liegen üblicherweise in Form einer Einzeldosis vor und können durch jedes der auf dem Gebiet der pharmazeutischen Technologie bekannten Verfahren hergestellt werden. Im wesentlichen enthalten alle Verfahren den Schritt des Zusammenbringens des aktiven Bestandteils mit dem Trägerstoff, gegebenenfalls mit einem oder mehreren zusätzlichen Bestandteilen. Im allgemeinen werden die Formulierungen durch gleichmäßiges und inniges Vermischen des aktiven Bestandteils mit einem flüssigen Träger oder einem fein verteilten festen Träger oder beiden und anschließend, falls erfoderlich, Formen des Produktes in die gewünschte Zubereitungsform, hergestellt.

Die erfindungsgemäßen Formulierungen für die orale Verabreichung können in Form von diskreten Einheiten, wie z.B. Kapseln, Cachets, Tabletten oder Pastillen, vorliegen, wobei jede Form eine bestimmte Menge des aktiven Bestandteils enthält. Sie können ebenfalls in Form eines Pulvers oder in Form eines Granulates oder in Form einer Lösung oder einer Suspension in einer wäßrigen oder nicht-wäßrigen Flüssigkeit, oder in Form einer öl-in-Wasser-Emulsion oder Wasser-in-Öl-Emulsion, vorliegen. Der aktive Bestandteil kann ebenfalls in Form eines Bolus, eines Elektuariums oder einer Paste vorliegen.

Wenn die erfindungsgemäßen Zubereitungen in Form einer Tablette vorliegen, kann man diese durch Verpressen oder Vergießen des aktiven Bestandteils gegebenenfalls zusammen mit einem oder mehreren zusätzlichen Bestandteilen herstellen. Verpreßte Tabletten kann man durch Pressen des aktiven Bestandteils in freifließender Form, z.B. als Pulver oder als Granulat, gegebenenfalls vermischt mit einem Bindemittel, einem Gleitmittel, einem inerten Verdünnungsmittel, oberflächenaktiven oder Dispersions-Mittel in einer geeigneten Vorrichtung herstellen. Gegossene Tabletten kann man durch Vergießen einer Mischung des in Pulverform vorliegenden aktiven Bestandteils und eines geeigneten Trägerstoffs, der mit einem inerten flüssigen Verdünnungsmittel befeuchtet ist, in einer geeigneten Vorrichtung herstellen.

Die erfindungsgemäßen Zubereitungen für die rektale Verabreichung können in Form von Suppositorien vorliegen, wobei der aktive Bestandteil in einem Träger, z.B. aus Kakaobutter, enthalten ist. Sie können auch in Form eines Klistiers vorliegen.

Wenn die erfindungsgemäßen Formulierungen für die parenterale Verabreichung vorgesehen sind, enthalten sie üblicherweise eine sterile wäßrige Zubereitung des aktiven Bestandteils, die vorzugsweise mit dem Blut des Empfängers isotonisch ist.

Erfindungsgemäße Zubereitungen, die für die intraartikuläre Verabreichung geeignet sind, können in Form einer sterilen wäßrigen Zubereitung des aktiven Bestandteils vorliegen, wobei der aktive Bestandteil gegebenenfalls in mikrokristalliner Form vorliegt, z.B. in Form einer wäßrigen mikrokristallinen Suspension.

Die erfindungsgemäßen Formulierungen können gleichfalls in Form einer liposomalen Zubereitung oder in Form eines bioabbaubaren Polymersystems zur Verabreichung des aktiven Bestandteils vorliegen.

Für die topische Verabreichung geeignete erfindungsgemäße Formulierungen enthalten flüssige oder halbflüssige Zubereitungen, wie z.B. Einreibemittel, Lotionen, Verbände, Öl-in-Wasser- oder Wasser-in-Öl-Emulsionen, wie z. B. Cremes, Salben oder Pasten, oder Lösungen oder Suspensionen, wie z. B. Tropfen. Zum Beispiel kann der aktive Bestandteil für die ophthalmologische Verabreichung in Form von wäßrigen Augentropfen, beispielsweise in Form einer 0,1 bis 1,0 %igen Lösung, vorliegen.

Für die Verabreichung durch die Nase oder in die Mundhöhle oder zur Inhalation geeignete erfindungsgemäße Formulierungen liegen in Form eines selbstzerstäubenden Pulvers oder in Form von Sprayzubereitungen, z. B. als Aerosol, vor. Die Formulierungen ergeben nach Dispersion vorzugsweise eine Teilchengröße im Bereich von 10 bis 200 µm, für die Inhalation vorzugsweise zwischen 1 und 100 µm.

Erfindungsgemäße Formulierungen können den aktiven Bestandteil auch in einer wäßrigen oder verdünnten alkoholischen Lösung enthalten. Der aktive Bestandteil kann gegebenenfalls durch ein Sprühgerät in einen feinen Nebel überführt werden, der von den Patienten inhaliert wird.

Derartige Zubereitungen enthalten üblicherweise ein Geschmacksmittel, wie z. B. Natriumsaccharin und ein ätherisches Öl. Ebenfalls kann eine Puffersubstanz und/oder ein oberflächenaktives Mittel in derartigen Zubereitungen zusammen mit Konservierungsmitteln, wie z.B. Methylhydroxybenzoat, enthalten sein.

Andere Zubereitungen, die zur Verabreichung durch die Nase geeignet sind, bestehen aus einem groben Pulver, das eine Teilchengröße von 20 bis 500 µm aufweist, das auf die gleiche Art und Weise wie Schnupftabak verabreicht wird.

Zusätzlich zu den vorhin genannten Bestandteilen können die erfindungsgemäßen Formulierungen einen oder mehrere weitere übliche und bekannte Komponenten, wie z.B. Verdünnungsmittel, Puffersubstanzen, Geschmacksstoffe, Bindemittel, oberflächenaktive Mittel, Verdickungsmittel, Gleitmittel, Konservierungsmittel, Antioxidantien, Emulgiermittel und dergleichen enthalten.

Die folgenden Beispiele erläutern die Erfindung ohne diese auf die Beispiele zu beschränken.

### Beispiel 1

a) Isolierung von Androsin aus pflanzlichem Material (Picrorhiza kurroa Royle und Benth).

Picrorhiza kurroa ist eine in Indien wachsende Pflanze mit röhrenförmigen Wurzeln.

Pulverisierte Wurzeln von Picrorhiza Kurroa werden einer erschöpfenden Soxhlet-Extraktion mit Lösungsmitteln steigender Polarität, z. B. Hexan-Chloroform-Ethylacetat-Methanol, unterworfen. Die Extrakte werden auf biologische Aktivität getestet, z. B. Einfluß auf die Histaminfreisetzung in Leukozyten. Die aktiven Fraktionen werden einer Gelfiltration (z. B. Sephadex^{R}-LH-20-Säule 2,5 x 78,5 cm) unterworfen. Als Eluierungsmittel wird Methanol verwendet. Androsinhaltige Fraktionen werden durch ihre biologische Aktivität, Test wie vorstehend beschrieben, identifiziert, vereinigt und zur Trockne eingedampft. (Der Methanolextrakt enthält etwa 1,6 % Androsin.)

b) Alternativ werden pulverisierte Wurzeln mit Hexan, anschließend mit Methanol einer Soxhlet-Extraktion unterworfen. Die Methanolfraktion wird, wie in a) beschrieben, weiter aufgetrennt und die aktiven Fraktionen durch ihre Aktivität in einem entsprechenden Testverfahren identifiziert.

Androsin kann dünnschichtchromatographisch identifiziert werden. Die Auftrennung erfolgt auf SiO₂-Platten mit einem Laufmittel aus Chloroform/Methanol, 8,5 : 1,5, und Detektion mit Vanillin-Schwefelsäure-Reagenz, wobei sich androsinhaltige Fraktionen nach Erhitzen auf 100 °C orangerot färben.

c) Verfahren zur Herstellung eines mit Androsin angereicherten Extraktes aus pflanzlichem Material

Die nach a) oder b) erhaltenen androsinhaltigen Fraktionen werden einer RP-Chromatographie (RP-18-Lobar-Säule) mit Methanol/Wasser als mobile Phase unterworfen. Mit 20 %igem Methanol werden die aktiven Wirkstoffe eluiert und anhand ihrer biologischen Aktivität identifiziert.

Die vorstehend beschriebenen Verfahren zur Isolierung von Androsin oder zur Herstellung eines mit Androsin angereicherten Extraktes umfassen im wesentlichen die erschöpfende Extraktion von pulverisierten Wurzeln von Picrorhiza Kurroa entweder mit Lösungsmitteln beginnend bei Hexan bis Methanol entsprechend der eluetropen Reihe durch Soxhlet-Extraktion, Perkulation, Maceration oder durch überkritische Gase (CO₂, Butan) und Anreichern der androsinhaltigen Fraktion bzw. Abtrennen der nicht androsinhaltigen Fraktion durch dem Fachmann bekannte säulenchromatographische Verfahren (z. B. Gelpermeationsverfahren, RP-Chromatographie) oder durch ein Flüssig/Flüssig-Verteilungsverfahren.

### Beispiel 2

### Herstellung von Verbindungen der allgemeinen Formel (I) auf synthetischem Weg

Eine Lösung von 10 mmol eines Acetophenons
der allgemeinen Formel 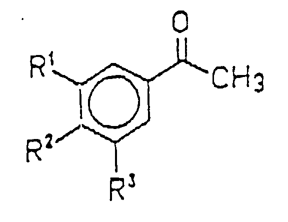 in der R¹ = CH₃O oder H, R² = H oder OH und R³ = H, OH oder CH₃O bedeutet, in 10 bis 15 ml Aceton wird mit 18 mmol von 2,5 %igem Kaliumhydroxid versetzt und bei Raumtemperatur 10 min gerührt. Anschließend wird eine Lösung von β-Acetobromglukose (14 bis 16 mmol) in 20 bis 30 ml Aceton langsam zugetropft und 24 bis 40 Stunden weitergerührt. Nach Neutralisierung des Reaktionsgemisches mit 10 %iger Salzsäure und Abkühlung auf ca. 0 °C wird der ausgefallene Niederschlag abgesaugt oder nach Eindampfen durch Extraktion mit Methylacetat isoliert. Die Umkristallisierung des Produktes aus Methanol oder Ethanol ergibt das kristalline Tetraacetylglucosyloxy-Acetophenon-Derivat mit einer Ausbeute von 20 bis 40 %.

Zu einer methanolischen Lösung von 2 mmol der im ersten Schritt erhaltenen Verbindung wird 1N Natriummethylat (9 mmol) gegeben und bei Raumtemperatur 2 bis 3 Stunden gerührt. Das Reaktionsgemisch wird mit Ionenaustauscherharz (IR-120) vorsichtig neutralisiert und im Vakuum eingedampft. Der Rückstand wird aus Wasser umkristallisiert und liefert das Glukosid als kristallines Produkt mit einer Ausbeute von 60 bis 70 %.

Zur Herstellung der halogenierten Derivate werden die Acetophenone (0,01 Mol) in Wasser bzw. in einem Wasser-Methanolgemisch gelöst und nach Zugabe von Na-Acetat (1,5 - 3,0 g) mit einer Lösung von Na-Hypochlorit (1,7 g) oder K-Iodid (0,5 g) und Iod (gasförmig) bzw. K-Bromid und Brom (gasförmig) bei - 60 °C bis 90 °C für 1,5 bis 3 Stunden umgesetzt (Cl, Br, I = 0,01 Mol). Das Reaktionsprodukt wird mit Chloroform ausgeschüttelt, mit Na-Thiosulfat gewaschen und die Rohprodukte aus Essigsäure umkristallisiert. Anschließend wird wie oben beschrieben weiterverarbeitet.

### Beispiel 3

### Biologische Untersuchungen

a) Hemmung der Histaminfreisetzung aus humanen peripheren Leukozyten durch verschiedene Lösungsmittelfraktionen Picrorhiza Kurrooa und Androsin. Von atopischen Spendern wurde venöses Blut entnommen und die peripheren Leukozyten durch Dextran-Sedimentation gewonnen. Die gereinigten Zellen wurden mit drei bzw. vier verschiedenen Konzentrationen der zu testenden Substanz sowie mit dem Lösungsmittel allein vorinkubiert. Nach 10 Minuten wurden die Zellen mit Kaninchenanti Human-IgE-Antikörper, Anaphylatoxin (C5a) enthaltend Plasma nach Vallota und Müller-Eberhard, Calcium-Ionophor oder Kochsalzlösung versetzt. 30 Minuten später wurde die Reaktion gestoppt und die Histaminkonzentration im Überstand spektrofluorometrisch gemessen.

Die Ergebnisse sind in Tabelle 1 dargestellt, wobei die Inhibierung der Histaminfreisetzung in Prozent der maximalen Freisetzung durch Perchlorsäure, korrigiert um die spontane Freisetzung ohne Stimulierung, angegeben ist.

b) Asthmaprotektive Wirkung der Acetophenonverbindung in Meerschweinchen.

Männliche weiße "Pirbright" Meerschweinchen wurden gegen Ovalbumin nach bekannten Verfahren sensibilisiert. Inhalationsversuche wurden 4 bis 6 Wochen später durchgeführt.

Spontan atmende Tiere wurden in einen Zwei-Kammer-Körper-Plethysmograph gegeben, wobei beide Kammern durch einen wassergefüllten Gummikragen um den Kopf der Tiere getrennt waren. Volumenänderungen in beiden Kammern wurden durch Drucküberträgersysteme gemessen. Der Grad der bronchialen Obstruktion wurde mittels des Parameters "compressed air" bestimmt. Diese Technik ist etwa 10 mal sensitiver als andere invasive Methoden (Dorsch et.al., Pfügers Arch., 1981, 391: 236-241).

Gruppen von 10 bis 14 Tieren wurden in zwei Untergruppen getrennt und entweder mit der Testsubstanz oder mit einer Kontroll-Lösung behandelt. 3 oder 4 Tage später wurden die Versuche wiederholt, wobei die Tiere, die vorher mit der Kontrolle behandelt wurden die Testsubstanz, und umgekehrt, erhielten (jedes Tier erhielt einmal die Testsubstanz oder das Lösungsmittel).

Ovalbumin, Histamin und Acetylcholin wurden in Kochsalzlösung gelöst; PAF wurde zuerst in Ethanol und anschließend in Kochsalzlösung, die 0,25 % Rinderserumalbumin enthielt, zu einer Endkonzentration von 1µg PAF pro ml gelöst. Ovalbumin, PAF, Histamin und Acetylcholin wurden in Form eines durch Ultraschall erzeugten Aerosols verabreicht.

Die Wirkung der Testsubstanz auf die PAF induzierte Hyper-Reaktivität wurde durch aufeinanderfolgende Inhalation von Histamin, PAF und Histamin untersucht. Eine Gruppe von Tieren wurde in zwei Untergruppen geteilt, wobei beide Untergruppen Histamin in einer Dosis inhalierten, die zu einer mittleren bronchialen Obstruktion führt. Eine Stunde später erhielt eine Gruppe die zu testende Substanz, die andere Gruppe erhielt das Lösungsmittel allein. Beide Gruppen wurden später durch die aufeinanderfolgende Inhalation von PAF und Histamin induziert. Zunächst wurde 1 µg PAF und 60 Minuten später die gleiche Histamin-Dosis wie vorher verabreicht. Die zweite Histamin-Kontaktierung führt in Kontrolltieren üblicherweise zu asthmatischen Reaktionen, die dreimal so stark sind wie die erste Kontaktierung.

Die Ergebnisse dieser Versuche zeigen die Abbildungen 1 und 2.

Wie man den Abbildungen entnehmen kann, reduziert Androsin in einer oralen Dosis von 10 mg/kg Körpergewicht das PAF-induzierte Asthma bronchiale in vivo um 72 % und das allergische Asthma bronchiale um 67 %. Als Aerosol verringerte Androsin in einer Dosis von 0,5 mg/Tier PAF-induzierte Reaktionen um 72 % und allergische Reaktionen um 67 %. Im wesentlichen die gleichen Wirkungen werden mit der Verbindung Apocynin und 2,4-Dimethoxy-2-hydroxyacetophenon erreicht.

Wie aus der Tabelle 1 ersichtlich ist, war in dem in-vitro-Versuch die Reinsubstanz Androsin nicht wirksam. Eine gute Hemmwirkung zeigten jedoch die androsinhaltigen Extrakte.

**Tabelle 1**

| Fraktion/Verbindung | Konzentration (mg/ml) | % Inhibierung der Histaminfreisetzung | | |
|---|---|---|---|---|
| | | α -IgE | C5a | A 12387 |
| Chloroform-Fr. | 0,1 | 56,2 ± 45,2 | 8,9 ± 51,6 | 76,0 ± 13,8 |
| | 0,01 | 50,2 ± 28,8 | 1,7 ± 63,7 | 8,7 ± 12,9 |
| | 0,001 | 35,3 ± 30,6 | 6,4 ± 35,2 | 9,0 ± 7,5 |
| | | | | |
| Ethylacetat-Fr. | 0,1 | 64,4 ± 35,9 | 38,3 ± 42,5 | 62,9 ± 28,4 |
| | 0,01 | 48,2 ± 44,4 | 24,9 ± 30,0 | 6,5 ± 20,9 |
| | 0,001 | -3,0 ± 44,8 | 20,8 ± 23,8 | 1,8 ± 13,6 |
| | | | | |
| Butanol-Fr. | 0,1 | 23,4 ± 59,4 | -13,9 ± 60,5 | 7,5 ± 41,6 |
| | 0,01 | 34,2 ± 52,9 | 13,3 ± 64,0 | -13,4 ± 29,7 |
| | 0,001 | 20,9 ± 56,7 | 32,1 ± 48,8 | -12,6 ± 18,1 |
| | | | | |
| Wasser-Fr. | 0,1 | 42,9 ± 49,9 | -20,0 ± 22,3 | 7,1 ± 40,2 |
| | 0,01 | 33,8 ± 56,9 | -11,0 ± 37,4 | -9,7 ± 15,4 |
| | 0,001 | 48,9 ± 49,9 | -14,9 ± 45,9 | -4,3 ± 15,1 |
| | | | | |
| Androsin | 0,1 | 10,9 ± 10,6 | -4,3 ± 26,7 | -12,5 ± 6,1 |
| | 0,01 | 5,5 ± 13,3 | 3,7 ± 16,3 | -14,9 ± 12,4 |
| | 0,001 | 0,5 ± 6,6 | -4,5 ± 28,2 | -0,4 ± 23,4 |
| | 0,0001 | 2,8 ± 9,0 | -5,4 ± 27,6 | -3,6 ± 23,3 |

## Patentansprüche

1. Verwendung einer Verbindung der Formel 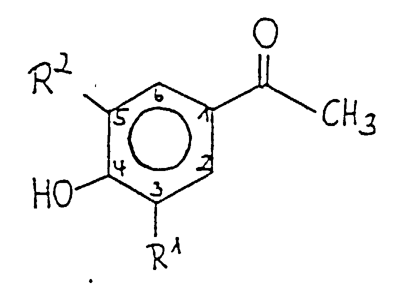 worin
R¹ = H, Cl, Br, I, Methyl, Hydroxy, Methoxy, Propoxy, Isopropoxy oder Ethoxy, und
R² = H, Cl, Br, I, Methyl, Hydroxy, Methoxy, Propoxy, Isopropoxy oder Ethoxy darstellen,
wobei R¹ und R² nicht gleichzeitig H bedeuten,
oder eines pharmazeutisch annehmbaren Ethers oder Esters davon in Kombination mit einem pharmazeutisch annehmbaren Träger zur Herstellung von Arzneimitteln zur Behandlung und/oder Prophylaxe des Asthma bronchiale, der obstruktiven Bronchitis oder allergischen Hauterkrankungen.

2. Verwendung nach Anspruch 1, worin in der Formel R¹ Methyl und R² Wasserstoff bedeutet.

3. Verwendung nach Anspruch 1, worin in der Formel R¹ die Gruppe n-Propoxy und R² Wasserstoff bedeutet.

4. Verwendung nach Anspruch 1, worin in der Formel R¹ die Gruppe Isopropoxy und R² Wasserstoff bedeutet.

5. Verwendung nach Anspruch 1, worin in der Formel R¹ und R² jeweils die Methoxygruppe bedeuten.

6. Verbindungen der allgemeinen Formel 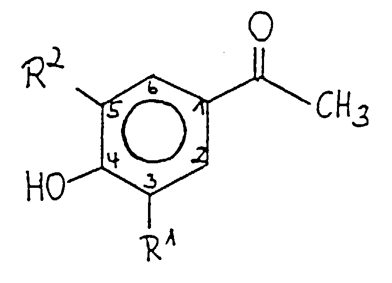 worin R¹ Methoxy und R² = Cl, Br oder I bedeuten,
sowie deren pharmazeutisch annehmbare Ester und Ether.

7. Verfahren zur Herstellung der halogenierten Verbindungen nach Anspruch 6, dadurch gekennzeichnet, daß man ein Acetophenon der allgemeinen Formel 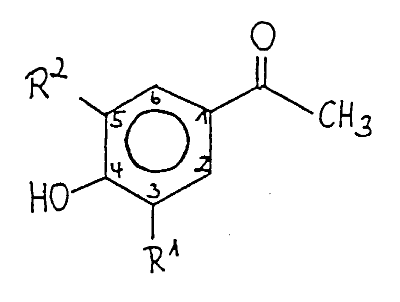 worin R¹ die Methoxygruppe und
R² Wasserstoff bedeuten,
a) in einem Wasser/Methanolgemisch löst,
b) Natriumacetat hinzufügt und mit einer Lösung von Natriumhypochlorit oder Kaliumjodid und Jod (gasförmig) oder Kaliumbromid und Brom (gasförmig) für 1,5 bis 3 Stunden bei - 60 °C bis 90 °C umsetzt,
c) das Reaktionsprodukt mit Chloroform ausschüttelt, mit Natriumthiosulfat wäscht und aus Essigsäure umkristallisiert und erforderlichenfalls zu dem gewünschten Ether oder Ester weiterverarbeitet.

8. Arzneimittel, dadurch gekennzeichnet, daß es eines oder mehrere Verbindungen der allgemeinen Formel nach Anspruch 6 enthält.

9. Verwendung einer Verbindung der allgemeinen Formel nach Anspruch 6 zur Herstellung von Arzneimitteln zur Behandlung und Prophylaxe von Asthma bronchiale, obstruktiver Bronchitis oder allergischen Erkrankungen.

## Claims

1. Use of a compound of the formula 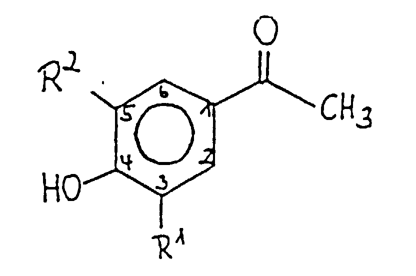 wherein
R¹ = H, Cl, Br, I, methyl, hydroxy, methoxy, propoxy, isopropoxy or ethoxy, and
R²=H, Cl, Br, I, methyl, hydroxy, methoxy, propoxy, isopropoxy or ethoxy,
whereby R¹ and R² do not mean H simultaneously,
or of a pharmaceutically acceptable ether or ester of it in combination with a pharmaceutically acceptable carrier for the manufacture of pharmaceutical preparations for the treatment and/or prophylaxis of bronchial asthma, obstructive bronchitis or allergic skin diseases.

2. Use according to Claim 1, wherein in the formula, R¹ means methyl and R² means hydrogen.

3. Use according to Claim 1, wherein in the formula, R¹ means the n-propoxy group and R² means hydrogen.

4. Use according to Claim 1, wherein in the formula, R¹ means the isopropoxy group and R² means hydrogen.

5. Use according to Claim 1, wherein in the formula, R¹ and R² each mean the methoxy group.

6. Compounds of the general formula 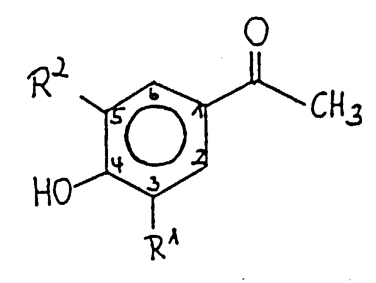 wherein R¹ means methoxy and R² = Cl, Br or I,
as well as their pharmaceutically acceptable esters and ethers.

7. Process for the manufacture of the halogenised compounds according to Claim 6, characterised in that an acetophenone of the general formula 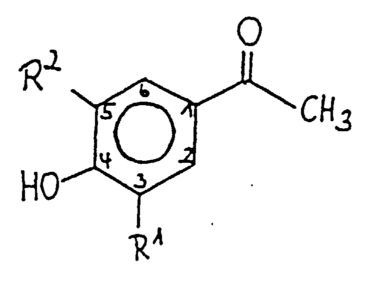 wherein R¹ means the methoxy group and R² means hydrogen,
a) is dissolved in a water/methanol mixture,
b) after adding sodium acetate is converted with a solution of sodium hypochlorite or potassium iodide and iodine (gaseous) or potassium bromide and bromine (gaseous) for 1.5 to 3 hours at - 60 °C to 90 °C,
c) and the resulting reaction product is extracted with chloroform, washed with sodium thiosulphate and recrystallised from ethanoic acid and where required is further processed to produce the desired ether or ester.

8. Pharmaceutical preparation, characterised in that it contains one or more compounds of the general formula according to Claim 6.

9. Use of a compound of the general formula according to Claim 6 for the manufacture of pharmaceutical preparations for the treatment and prophylaxis of bronchial asthma, obstructive bronchitis or allergic diseases.

## Revendications

1. Mise en oeuvre d'un dérivé de formule 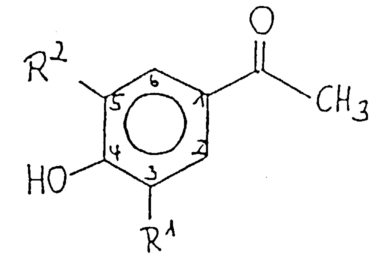 dans laquelle
R¹ désigne H, CI, Br, I, un groupe méthyle, hydroxy, méthoxy, propoxy, isopropoxy ou éthoxy, et
R² désigne H, CI, Br, I, un groupe méthyle, hydroxy, méthoxy, propoxy, isopropoxy ou éthoxy,
R¹ et R² ne désignant pas simultanément H,
ou d'un éther ou d'un ester pharmaceutiquement acceptable de ce dérivé combiné à un véhicule pour la préparation de médicaments destinés au traitement et/ou à la prévention de l'asthme bronchique, de la bronchite obstructive ou de dermatoses allergiques.

2. Mise en oeuvre selon la revendication 1, R¹ désignant un groupe méthyle et R² de l'hydrogène dans la formule.

3. Mise en oeuvre selon la revendication 1, R¹ désignant le groupe n-propoxy et R² de l'hydrogène dans la formule.

4. Mise en oeuvre selon la revendication 1, R¹ désignant le groupe isopropoxy et R² de l'hydrogène dans la formule.

5. Mise en oeuvre selon la revendication 1, R¹ et R² désignant chacun le groupe méthoxy dans la formule.

6. Dérivés de formule générale 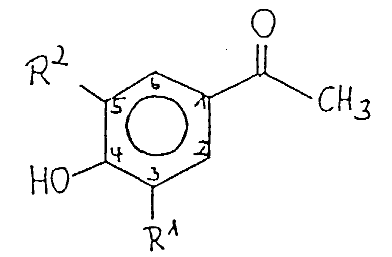 dans laquelle R¹ désigne un groupe méthoxy et R² Cl, Br ou I, ainsi que leurs esters ou éthers pharmaceutiquement acceptables.

7. Procédé pour la préparation de dérivés halogénés selon la revendication 6, caractérisé en ce qu'une acétophénone de formule générale 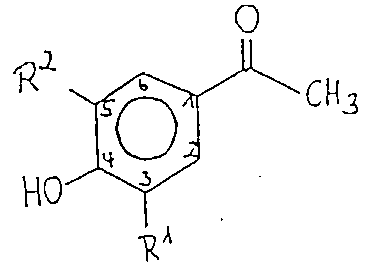 dans laquelle R¹ désigne le groupe méthoxy et
R² de l'hydrogène,
a) est dissoute dans un mélange eau/méthanol,
b) on ajoute de l'acétate de sodium et on le convertit avec une solution d'hypochlorite de sodium ou d'iodure de potassium et d'iode (gazeux) ou de bromure de potassium et de brome (gazeux), pendant 1,5 à 3 heures, à une température de l'ordre de -60 à 90 °C,
c) le produit réactionnel est agité avec du chloroforme, lavé avec du thiosulfate de sodium et recristallisé à partir d'acide acétique avant de subir, au besoin, un traitement secondaire pour obtenir l'éther ou l'ester recherché.

8. Médicament caractérisé en ce qu'il contient un ou plusieurs des dérivés de formule générale selon la revendication 6.

9. Mise en oeuvre d'un dérivé de formule générale selon la revendication 6 pour préparer des médicaments destinés au traitement et à la prévention de l'asthme bronchique, de la bronchite obstructive ou d'affections allergiques.
